# FASCICULE DE BREVET EUROPEEN

(11) **EP 0 688 788 B1**
(45) Date de publication et mention de la délivrance du brevet: **21.03.2001**
(21) Numéro de dépôt: 95401462.7
(22) Date de dépôt: 21.06.1995
(51) Int. Cl.: C07K 5/065, C07F 5/02, A61K 38/05, A61K 33/22

(54) **Dérivés peptidiques de l'acide boronique ayant une activité inhibitant de protéase, leur procédé de préparation et les composition et les compositions pharmaceutiques les contenants**
Peptidderivate der Boronsäure mit Proteasen inhibierender Aktivität, ihr Herstellungsverfahren und sie enthaltende pharmazeutische Zubereitungen
Boronic acid peptide derivatives having protease inhibiting activity, process for preparation thereof and pharmaceutical compositions comprising them

(30) Priorité: 22.06.1994 FR 9407589
(43) Date de publication de la demande: 27.12.1995
(73) Titulaire: ADIR ET COMPAGNIE, 92415 Courbevoie Cédex (FR)
(72) Inventeur: de Nanteuil, Guillaume, F-92150 Suresnes (FR); Lila, Christine, F-78220 Viroflay (FR); Gloanec, Philippe, F-78170 La Celle Saint Cloud (FR); Laubie, Michel, F-92420 Vaucresson (FR); Verbeuren, Tony, F-78540 Vernouillet (FR); Simonet, Serge, F-78700 Conflans Sainte Honorine (FR); Rupin, Alain, F-37510 Savonnieres (FR)

(56) Documents cités:
- EP-A- 0 293 881
- EP-A- 0 471 651
- EP-A- 0 615 978
- WO-A-92/07869
- WO-A-94/25049
- WO-A-95/09634
- J. MED. CHEM. (1993), 36(13), 1831-8 CODEN: JMCMAR;ISSN: 0022-2623, LIM, MARGUERITA S. L. ET AL 'The solution conformation of (D)Phe-Pro-containing peptides: implications on the activity of Ac-(D)Phe-Pro-boroArg-OH, a potent thrombin inhibitor'
- TETRAHEDRON LETT. (1992), 33(29), 4209-12 CODEN: TELEAY;ISSN: 0040-4039, ELGENDY, SAID ET AL 'New peptide boronic acid inhibitors of thrombin'

## Description

La présente invention concerne des dérivés peptidiques d'acide boronique, leur procédé de préparation, les compositions pharmaceutiques qui les contiennent ainsi que leur utilisation en tant qu'inhibiteurs de trypsine-like serine proteases.

L'une de ces serine proteases, la thrombine, est l'enzyme clé de la coagulation et joue un rôle central dans la pathologie des thromboses veineuses et artérielles comme l'ont montré F. Toti et coll. (Sang, Thrombose, Vaisseaux, 4, 483-494, 1992) et T.M. REILLY et coll. (Blood Coagulation and Fibrinolysis, 3, 513-517, 1992).

Les approches anti-thrombotiques sont plus efficaces et sans risque par rapport aux traitements actuels. Des inhibiteurs directs de la thrombine, actuellement en développement clinique, présentent toute une série d'avantages sur l'héparine. Mais ces substances, l'hirudine et l'hirulog-1 ont le désavantage de ne pas être actives par voie orale.

D'autre part, il est connu que des peptides contenant la séquence (D)Phe-Pro-Arg sont des inhibiteurs du site catalytique de la thrombine (C. KETTNER et coll., J. Biol. Chem., 265 (30), 18289-18297, 1990).

Des dérivés peptidiques de l'acide boronique, présentant une activité anti-thrombotique, ont déjà été décrits dans la littérature. C'est le cas plus particulièrement des composés décrits dans les brevets EP 293881 EP 615978 et EP 471651. M.A. HUSSAIN et coll. ont d'ailleurs démontré que l'acide Ac-(D)Phe-Pro-Arg boronique (DUP 714) est un inhibiteur de la thrombine (Peptides, 12, 1153-1154, 1991).

Il était donc particulièrement intéressant de synthétiser de nouveaux inhibiteurs de serine proteases afin d'augmenter la puissance, la selectivité ainsi que l'activité par voie orale des composés déjà décrits dans la littérature.

Plus spécifiquement, la Drésente invention concerne les comDosés de formule (I) : 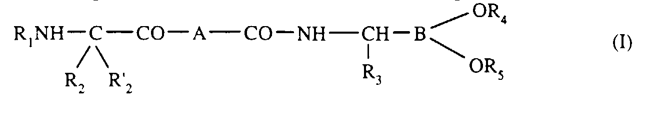 dans laquelle :
- R₁: représente un atome d'hydrogène, un groupement acyle (C₁-C₆) linéaire ou ramifié, alkoxycarbonyle (C₁-C₆) linéaire ou ramifié, benzyloxycarbonyle, phénoxycarbonyle, alkyle (C₁-C₆) linéaire ou ramifié (substitué ou non par un ou plusieurs groupements phényle, carboxy, alkoxycarbonyle (C₁-C₆) linéaire ou ramifié, phénoxycarbonyle, benzyloxycarbonyle ou morpholinosulfonyle) ou un groupement R₆SO₂- dans lequel R₆ représente un groupement alkyle (C₁-C₆) linéaire ou ramifié, naphtyle, phényle, benzyle, morpholine (chacun des groupements naphtyle, phényle ou benzyle étant lui-même éventuellement substitué par un ou plusieurs atomes d'halogène ou groupements alkyle (C₁-C₆) linéaire ou ramifié, alkoxy (C₁-C₆) linéaire ou ramifié, trihalogénométhyle, amino, alkylamino ou dialkylamino),
- R₂: représente un atome d'hydrogène ou un groupement :
- phényle,
- benzyle (substitué ou non sur le noyau phényle par un ou plusieurs atomes d'halogène ou groupements alkyle (C₁-C₆) linéaire ou ramifié, alkoxy (C₁-C₆) linéaire ou ramifié, hydroxy, amino, nitro ou carboxy),
- thiénylméthyle,
- (pyridinyl)méthyle,
- diphénylméthyle,
- fluorényle,
- naphtylméthyle,
- benzocyclobutyle,
- (dicyclopropylméthyl)méthyle,
- indanyle,
- ou, cycloalkyl (C₃-C₇) méthyle,
- R'₂: représente un atome d'hydrogène
- R₃: représente l'un quelconque des groupements suivants : 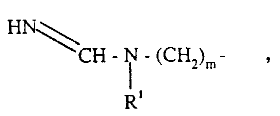 , 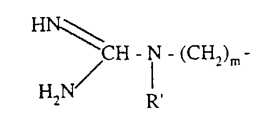 ou, 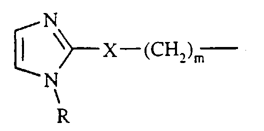 dans lesquels :
1 ≤ m ≤ 6,
R représente un atome d'hydrogène ou un groupement alkyle (C₁-C₆) linéaire ou ramifié,
R' représente un groupement alkyle (C₁-C₆) linéaire ou ramifié,
X représente un atome de soufre ou un groupement amino,
- R₄ et R₅: représentent chacun un atome d'hydrogène ou un groupement alkyle (C₁-C₆) linéaire ou ramifié,
ou 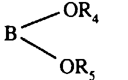 forme un ester boronique de pinanediol,
- A: représente le groupement suivant : 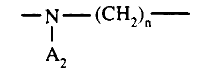 dans lequel :
n représente 1 ou 2,
A₂ représente un groupement phényle, indanyle, cycloalkyle (C₃-C₇) (substitué ou non par un ou plusieurs groupements alkyle (C₁-C₆) linéaire ou ramifié) cycloalkényle (C₃-C₇), bicyclo[2.1.1]hexyl, bicyclo[2.2.1]heptyl ou un groupement : 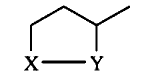 dans lequel
X et Y différents représentent un atome d'oxygène, de soufre, un groupement NH ou CH₂,
leurs énantiomères, diastéréoisomères et épimères ainsi que leurs sels d'addition à un acide ou à une base pharmaceutiquement acceptable.

L'invention concerne également le procédé de préparation des dérivés de formule (I) caractérisé en ce que l'on fait réagir un amino-acide protégé de formule (II), dont on a éventuellement séparé les isomères par une technique classique de séparation : 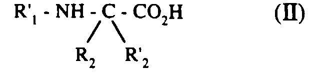 dans laquelle :
- R'₁: représente un groupement acyle (C₁-C₆) linéaire ou ramifié, benzyle, alkoxycarbonyle (C₁-C₆) linéaire ou ramifié, benzyloxycarbonyle ou phénoxycarbonyle,
- R₂ et R'₂: ont la même signification que dans la formule (I),
que l'on fait réagir selon la technique de couplage peptidique décrite par W. KONIG et R. GEIGER (Ber., 103, 788, 1970), avec un deuxième amino-acide protégé de formule (III) dont on a éventuellement séparé les isomères selon une technique classique de séparation,

HA - CO₂CH₂C₆H₅ (III)

dans laquelle A a la même signification que dans la formule (I), pour conduire au composé de formule (IV) : 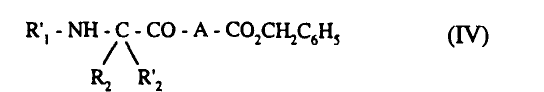 dans laquelle R'₁, R₂, R'₂ et A ont la même signification que précédemment,
dont on déprotège la fonction acide par hydrogénation catalytique ou saponification, pour conduire au composé de formule (V) : 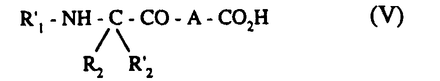 dans laquelle R'₁, R₂, R'₂ et A ont la même signification que précédemment,
que l'on met en réaction avec du N-hydroxysuccinimide en présence de 1,3-dicyclohexylcarbodiimide en milieu anhydre, pour conduire au composé de formule (VI) : 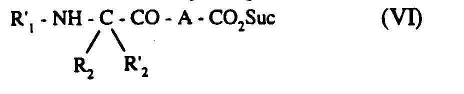 dans laquelle R'₁, R₂, R'₂ et A ont la même signification que précédemment et Suc représente un radical succinimido,
que l'on met en réaction en milieu basique avec un composé de formule (VII) : 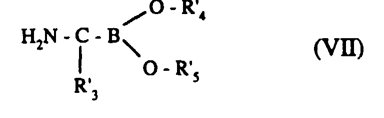 dans laquelle :
R'₃ représente le groupement Br-(CH₂)ₘ- dans lequel m a la même signification que dans la formule (I),
R'₄ et R'₅ représente chacun un groupement alkyle (C₁-C₆) linéaire ou ramifié,
ou 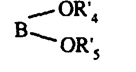 forme un ester boronique de pinanediol,
pour conduire au composé de formule (VIII) : 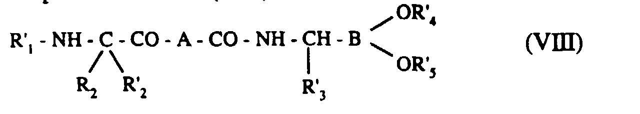 dans laquelle R'₁, R₂, R'₂, R'₃, A, R'₄ et R'₅ ont la même signification que précédemment, que l'on fait réagir avec de la thiourée éventuellement substituée ou un composé permettant d'accéder au dérivé aminé convenablement substitué,
pour conduire au composé de formule (I/a), cas particulier des composés de formule (I), 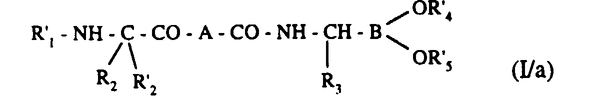 dans laquelle R'₁, R₂, R₃, R'₂, A, R'₄, R'₅, R et m ont la même signification que précédemment,
composé de formule (I/a) dont on déprotège, si on le souhaite, la fonction amine terminale et que l'on transforme, en milieu inerte, à l'aide de trichlorure de bore, en acide boronique de formule (I/b), cas particulier des composés de formule (I) : 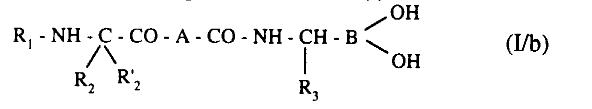 dans laquelle R₁, R₂, R'₂, A et R₃ ont la même signification que dans la formule (I),
composé de formule (I/a) ou (I/b) :
- que l'on purifie éventuellement selon une technique classique de purification,
- dont on sépare, si on le souhaite, les énantiomères selon une technique classique de séparation
et que l'on transforme, le cas échéant, en ses sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, oxalique, etc...

Parmi les bases pharmaceutiquement acceptables, on peut citer à titre non limitatif, l'hydroxyde de sodium, l'hydroxyde de potassium, le bicarbonate de sodium, etc...

Les composés de formule (VII) peuvent être obtenus à partir du composé de formule (IX) obtenu selon le procédé décrit par M.W. RATHKE et coll. (J. Organomet.. Chem., 122, 145-149, 1976) : 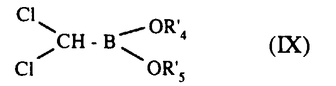 dans laquelle R'₄ et R'₅ sont tels que définis plus haut,
que l'on fait réagir sur un organomagnésien de formule (X) :

R'₃MgCl (X)

dans laquelle R'₃ a la même signification que précédemment,
pour conduire au composé de formule (XI) : 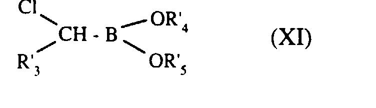 dans laquelle R'₃, R'₄ et R'₅ sont tels que définis précédemment, que l'on met en réaction avec le 1,1,1,3,3,3-hexaméthyldisilazane (HMDS) en présence de n-butyllithium pour conduire, après traitement en milieu acide, au composé de formule (VII).

Le composé de formule (XI) peut également être obtenu selon le procédé décrit par D.S. MATTESON et coll. (Organometallics, 3, 1284-1288, 1984) et W. RATHKE et coll. (J. Biol. Chem., 265 (30), 18289-18297, 1990).

Les composés de la présente invention, outre le fait qu'ils soient nouveaux, présentent des propriétés pharmacologiques particulièrement intéressantes.

Ce sont de puissants inhibiteurs de trypsine-like serine proteases qui présentent une importante selectivité vis-à-vis de la thrombine par rapport à d'autres serine proteases de la coagulation. Ils possèdent d'autre part une meilleure activité par voie orale que le composé de référence DUP 714.

Ces propriétés les rendent donc utiles dans le traitement des angines stables ou non, des maladies d'origine thrombotique et/ou donnant lieu à des complications thrombotiques ainsi que dans le traitement ou la prévention de l'infarctus du myocarde et des thromboses veineuses ou artérielles.

Ils peuvent également être utilisés en association thérapeutique avec un thrombolytique.

L'invention s'étend aussi aux compositions pharmaceutiques renfermant comme principe actif au moins un composé de formule (I) avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, etc...

La posologie utile est adaptable selon la nature et la sévérité de l'affection, la voie d'administration ainsi que selon l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits de départ connus ou préparés selon des modes opératoires connus.

La préparation A conduit à un intermédiaire de synthèse utile dans la préparation des composés de l'invention.

Les préparations B et C illustrent les procédés de préparation des composés de l'invention.

Les structures des composés décrits dans les exemples et celles de leurs intermédiaires ont été confirmées par les techniques spectroscopiques usuelles.

### Préparation A : (R)-1-Amino-4-bromobutylboronate de (+)α-pinanediol, chlorhydrate

Ce composé a été obtenu selon le procédé décrit par C. KETTNER et coll. (J. Biol. Chem., 265 (30), 18289-18297, 1990) par réaction du bromure d'allyle sur le catécholborane, suivie d'une transestérification avec du (+)α-pinanediol puis d'une réaction d'homologation en présence de dichlorométhyllithium et enfin de la réaction avec de l'hexaméthyl-disilazane.
Point de fusion : 160°C
Pouvoir rotatoire : [α]_{D}²⁵ = + 16,5° (c = 1 %, éthanol)

| Microanalyse élémentaire : | | | | |
|---|---|---|---|---|
| | C % | H % | N % | Cl % |
| calculé | 45,88 | 7,15 | 3,82 | 9,67 |
| trouvé | 45,82 | 7,09 | 4,13 | 9,85 |

Les abréviations utilisées dans les exemples sont les suivantes :
- Ac: représente acétyl,
- Fmoc: représente 9-fluorénylméthoxycarbonyl,
- Bz: représente benzyl,
- Suc: représente le groupement succinimido,
- (R)Phe: représente le résidu (R)-phénylalanyl,
- Gly: représente le résidu glycyl,
- Boc: représente t.butoxycarbonyl.

### Préparation B :1 -(R)-{[Ac-(R)Phe-(N-cyclopentyl)Gly]amino}-4-(isothiouréïdo) butylboronate de (+)α-pinanediol

### Stade A : Boc-(R)Phe-(N-cyclopentyl)Gly-OBz

En utilisant la technique de couplage peptidique (DCC/HOBT) décrite par W.KONIG et R.GEIGER (Ber., 103, 788, 1970) et le diméthylformamide anhydre comme solvant, le produit attendu est préparé à partir de 73 mmoles de (N-cyclopentyl)Gly-OBz et de Boc-(R)Phe-OH et est purifié par chromatographie sur colonne de silice en utilisant comme éluant un mélange dichlorométhane/éthanol (97/3).
Rendement : 76 %

### Stade B : H-(R)Phe-(N-cyclopentyl)Gly-OBz

Le produit obtenu au stade précédent est déprotégé par dissolution de 31 mmoles dans 150 ml d'acétate d'éthyle anhydre refroidi dans un bain eau-glace en réalisant un barbotage d'acide chlorhydrique gaz pendant une heure. Après retour à température ambiante et agitation pendant une heure, le mélange est évaporé. Le résidu est repris à l'éther et à nouveau évaporé.
Rendement : 99 %

### Stade C : Ac-(R)Phe-(N-cyclopentyl)Gly-OBz

5 mmoles du composé obtenu au stade précédent dans un mélange contenant 10 ml de dioxane, 5 ml d'eau, 24 ml d'anhydride acétique et 25 mmoles de bicarbonate de sodium sont agitées pendant 3 heures à température ambiante. Après évaporation, le résidu est repris par un mélange eau/acétate d'éthyle. La phase organique est lavée à l'eau, puis par une solution saturée de chlorure de sodium. Après séchage et évaporation, on obtient le produit attendu.
Rendement : 89 %

### Stade D : Ac-(R)Phe-(N-cyclopentyl)Gly-OH

5 mmoles du produit obtenu au stade précédent dans 25 ml de méthanol sont hydrogénées sous une pression d'hydrogène de 4 kg pendant 12 h en présence de 150 mg de palladium/C à 10 % anhydre. Après filtration du catalyseur, le produit attendu est obtenu après évaporation du solvant.

### Stade E : Ac-(R)Phe-(N-cyclopentyl)Gly-OSuc

A 4,46 mmoles de N-hydroxysuccinimide dans 50 ml de dichlorométhane anhydre, sont ajoutées 4,46 mmoles du produit obtenu au stade précédent dans 20 ml de dichlorométhane anhydre puis 4,46 mmoles de dicyclohexylcarbodiimide dissous dans du dichlorométhane. L'ensemble est agité 12 heures à température ambiante. Après filtration de la dicyclohexylurée formée, le produit attendu est obtenu après évaporation.

### Stade F : 1-(R)-{[Ac-(R)Phe-(N-cyclopentyl)Gly]amino}-4-bromobutylboronate de (+)α-pinanediol

4 mmoles du composé obtenu dans la préparation A dans 10 ml de dichlorométhane anhydre et 4 mmoles du composé obtenu au stade précédent sont placées sous atmosphère d'argon à -20°C. 56 ml de triéthylamine sont alors ajoutés goutte à goutte et l'ensemble est maintenu 30 minutes à -20°C. Après retour à température ambiante, le mélange est agité une nuit sous atmosphère d'argon. Après reprise par de l'acétate d'éthyle, lavage à l'eau, au bicarbonate de sodium, à l'eau, à l'acide chlorhydrique 0,2N et enfin à l'eau, la phase organique est séchée et évaporée. Le produit attendu est obtenu après purification sur résine "Sephadex^{®}".
Rendement : 90 %

### Stade G : 1-(R)-{ [Ac-(R)Phe-(N-cyclopentyl)Gly]amino}-4-(isothiouréïdo) butylboronate de (+)α-pinanediol

2,4 mmoles du composé obtenu au stade précédent et 7,3 mmoles de thiourée dans 6 ml d'éthanol sont agitées 60 heures à température ambiante. Après évaporation du solvant, le produit attendu est obtenu après purification par passage sur résine "Sephadex^{®}" en utilisant le méthanol comme éluant.
Rendement : 85 %

### Préparation C: Acide 1-(R)-{[Ac-(R)Phe-(N-cyclopentyl)Gly]amino}-4-(isothiouréïdo)butyl boronique, acétate

2 mmoles du composé obtenu dans l'exemple 1 dans 25 ml de dichlorométhane anhydre sont refroidies à -78°C sous atmosphère d'argon. 8 mmoles de trichlorure de bore sont alors additionnées goutte à goutte en 30 minutes. La température est amenée à 0°C et l'ensemble agité 30 minutes. 10 ml d'eau glacée sont alors ajoutés et après 15 minutes d'agitation, le mélange est amené à température ambiante. La phase organique est décantée, extraite par 10 ml d'un mélange eau/acide acétique (90/10). La phase aqueuse restante est lavée à l'éther et les phases aqueuses réunies sont évaporées. Le résidu est purifié sur Bio-gel en utilisant comme éluant un mélange eau/acide acétique (90/10) et conduit au produit attendu qui est lyophilisé. L'analyse physicochimique du produit est compatible avec la structure attendue.
Spectre de masse : FAB⁺: [M+glycérol-2H₂O+H⁺]: m/z = 561

### Exemple 7 :1-(R)-{[Ac-(R)Phe-(N-cyclopentyl)Gly]amino}-4-(1N-méthylguanidino) butyl boronate de (+)α-pinanediol

### Stades A à F : Ces stades sont identiques aux stades A à F de la préparation B.

### Stade G : 1-(R)-{[Ac-(R)Phe-(N-cyclopentyl)Gly]amino}-4-azidobutyl boronate de (+)α-pinanediol

### Stade H : 1-(R)-{[Ac-(R)Phe-(N-cyclopentyl)Gly]amino}-4-aminobutyl boronate de (+)α-pinanediol

Les produits attendus aux stades G et H sont obtenus selon les procédés décrits dans le brevet EP 615978.

### Stade I: 1-(R)-{[Ac-(R)Phe-(N-cyclopentyl)Gly]amino}-4-(N-méthylamino)butyl boronate de (+)α-pinanediol, benzène sulfonate

A 1 mmole du composé obtenu au stade précédent dans 20 ml d'éthanol anhydre sont ajoutés 5,7 g de tamis moléculaire 3Å et 3,75 ml d'une solution aqueuse de formaldéhyde à 40 %. L'ensemble est agité une nuit à température ambiante. Après filtration, 1 mmole d'acide benzène sulfonique est ajoutée aux phases éthanoliques et l'ensemble est hydrogéné en présence de 100 mg de Pd/C à 10 % comme catalyseur pendant une nuit à température ambiante et pression atmosphérique. Le produit attendu est obtenu après filtration du catalyseur et purification sur résine séphadex^{®}.

### Stade J : 1-(R)-{[Ac-(R)Phe-(N-cyclopentyl)Gly]amino}-4-(1N-méthylguanidino)butyl boronate de (+)α-pinanediol

Le produit attendu est obtenu par réaction du composé décrit au stade précédent avec du cyanamide selon le procédé décrit dans l'exemple 2 du brevet EP 615978.

### Exemple 8 : Acide 1-(R)-{[Ac-(R)Phe-(N-cyclopentyl)Gly]amino}-4-(1N-méthyl guanidino)butyl boronique, acétate

Le produit attendu est obtenu selon le procédé décrit dans la préparation C à partir du composé décrit dans l'exemple 7.
Spectre de masse : FAB⁺: [M+glycérol-2H₂O+H⁺]: m/z = 559

### Exemple 9 : 1-(R){[Ac-(R)Phe-(N-cyclohexyl)Gly]amino}-4-(1N-méthylguanidino) butyl boronate de (+)α-pinanediol

Le produit attendu est obtenu selon le procédé décrit dans l'exemple 7 en utilisant comme produit de départ la (N-cyclohexyl)Gly-OBz.

### Exemple 10 : Acide 1-(R)-{[Ac-(R)Phe-(N-cyclohexyl)Gly]amino}-4-(1N-méthyl guanidino)butyl boronique, acétate

Le produit attendu est obtenu selon le procédé décrit dans la préparation C à partir du composé décrit dans l'exemple 9.
Spectre de masse : FAB⁺: [I⁺]: [M+H]⁺ : m/z = 516

### Exemple 11 : 1-(R)-{[Ac-(R)(3-amino)Phe-(N-cyclopentyl)Gly]amino}-4-(1N-méthyl guanidino)butyl boronate de (+)α-pinanediol

### Stade A : (R,S) (3-Nitro)Phe-OH, chlorhydrate

Le chlorhydrate de 3-nitrophénylalanine est obtenu en faisant réagir le bromure de 3-nitrobenzyle avec l'acétamidomalonate d'éthyle en milieu éthanol anhydre puis en hydrolysant le malonate de diéthyle formé par de l'acide chlorhydrique 6N en présence d'acide acétique.

### Stade B : Fmoc-(R)(3-nitro)Phe-OH

A 4 mmoles du composé obtenu au stade précédent dissoutes dans 14,4 ml d'une solution aqueuse de carbonate de sodium à 10 % sont ajoutées après refroidissement 4 mmoles de Fmoc-Cl. Après retour à température ambiante, l'ensemble est agité une nuit puis versé sur 250 ml d'eau. La phase aqueuse est lavée à l'éther et acidifiée par de l'acide chlorhydrique concentré jusqu'à pH 1. Le produit attendu sous forme racémique est filtré, séché et les isomères sont séparés par HPLC sur colonne préparative DAICEL OD en utilisant comme éluant un mélange heptane/isopropanol/acide trifluoroacétique (650/350/0,5).

### Stade C : (R)(3-Nitro)Phe-OH

Le produit obtenu au stade précédent est déprotégé en milieu dioxane en présence de pipéridine.

### Stade D : Boc-(R)(3-nitro)Phe-OH

Le produit obtenu au stade précédent est protégé classiquement en présence de di.t.butylcarbonate.

### Stade E : Boc-(R)(3-nitro)Phe-(N-cyclopentyl)Gly-OBz

Le produit attendu est obtenu selon le procédé décrit au stade A de l'exemple 1 à partir du produit obtenu au stade précédent.

Stades F à M : Les produits attendus à ces stades sont obtenus selon les procédés décrits aux stades B à J de l'exemple 7.

### Exemple 12 : Acide 1-(R)-{[Ac-(R)(3-amino)Phe-(N-cyclopentyl)Gly]amino}-4-(1N méthylguanidino)butyl boronique, acétate

Le produit attendu est obtenu selon le procédé décrit dans la préparation C à partir du composé décrit dans l'exemple 11.
Spectre de masse : FAB⁺: [I⁺]: [M+M]⁺: m/z = 517

### Exemple 13 : 1-(R)-{[Morpholinosulfonyl-(R)Phe-(N-cyclopentyl)Gly]amino}-4-(1N méthylguanidino)butyl boronate de (+)α-pinanediol

Le produit attendu est obtenu selon le procédé décrit dans l'exemple 7 en remplaçant au stade A la Boc-(R)Phe-OH par la morpholinosulfonyl-(R)-Phe-OH décrite dans J. Med. Chem. (Vol. 34, n° 7, p. 1937, 1991).

### Exemple 14 : Acide 1-(R)-{[Morpholinosulfonyl-(R)Phe-(N-cyclopentyl)Gly]amino}-4 (1N-méthylguanidino)butyl boronique, acétate

Le produit attendu est obtenu selon le procédé décrit dans la préparation C à partir du composé décrit dans l'exemple 13.

### Exemple 15 : 1-(R)-{[Ac-(R)Phe-(N-cyclopentyl)Gly]amino}-4-(formimidoylamino)butyl boronate de (+)α-pinanediol

Stades A à H : Ces stades sont identiques aux stades A à H de l'exemple 7.

### Stade I: 1-(R)-{[Ac-(R)Phe-(N-cyclopentyl)Gly]amino}-4-(formimidoylamino)butyl boronate de (+)α-pinanediol

Le produit attendu est obtenu en faisant réagir le chlorhydrate d'éthyl formimidate sur le produit obtenu au stade précédent selon le procédé décrit dans le brevet PCT/US94/04058.

### Exemple 16 : Acide 1-(R)-{[Ac-(R)Phe-(N-cyclopentyl)Gly]amino}-4-(formimidoyl amino)butyl boronique, acétate

Le produit attendu est obtenu selon le procédé décrit dans la préparation C à partir du composé décrit dans l'exemple 15.
Spectre de masse : FAB⁺: [M+glycérol-2H₂O+H⁺]: m/z = 544

### Exemple 17 : 1-(R)-{[Ac-(R)Phe-(N-cyclopentyl)Gly]amino}-4-(N-méthyl-N formimidoylamino)butyl boronate de (+)α-pinanediol

Stades A à I : Ces stades sont identiques aux stades A à I de l'exemple 7.

### Stade J : 1-(R)-{[Ac-(R)Phe-(N-cyclopentyl)Gly]amino}-4-(N-méthyl-N-formimidoylamino)butyl boronate de (+)α-pinanediol

Le produit attendu est obtenu selon le procédé décrit au stade I de l'exemple 15 à partir du composé décrit au stade précédent.

### Exemple 18 : Acide 1-(R)-{[Ac-(R)Phe-(N-cyclopentyl)Gly]amino)-4-(formimidoyl amino)butyl boronique, acétate

Le produit attendu est obtenu selon le procédé décrit dans la préparation C à partir du composé décrit dans l'exemple A.
Spectre de masse : FAB⁺: [M+glycérol-2H₂O+H⁺] : m/z = 545

### Exemple 19 : 1-(R)-{[Ac-(R)Phe-(N-cyclopentyl)Gly]amino}-4-[(imidazol-2-yl)amino] butyl boronate de (+)α-pinanediol

Le produit attendu est obtenu selon le procédé décrit dans la préparation B en remplaçant au stade G la thiourée par le 1-trityl-2-aminoimidazole et en réalisant ensuite une hydrolyse acide.

### Exemple 20 : Acide 1-(R)-{[Ac-(R)Phe-(N-cyclopentyl)Gly]amino}-4-[(imidazol-2-yl) amino}butyl boronique, acétate

Le produit attendu est obtenu selon le procédé décrit dans la préparation C à partir du composé décrit dans l'exemple 19.
Spectre de masse : FAB⁺: [M+glycérol-2H₂O+H⁺]: m/z = 555

### Exemple 21 : 1 -(R)-{[Ac-(R)Phe-(N-cyclopentyl)Gly]amino}-4-[(1-méthylimidazol-2 yl)thio]butyl boronate de (+)α-pinanediol

Stades A à F : Ces stades sont identiques aux stades A à F de la préparation B.

### Stade G : 1-(R)-{ [Ac-(R)Phe-(N-cyclopentyl)Gly]amino}-4-[(1-méthylimidazol-2-yl) thio]butyl boronate de (+)α-pinanediol

A une solution refroidie à 0°C contenant 2 mmoles d'hydrure de sodium et 2 mmoles de l-méthyl-2-mercaptoimidazole dans 10 ml de diméthylformamide sont ajoutées 2 mmoles du dérivé bromé obtenu au stade F. Après retour à température ambiante, addition d'eau, extraction à l'acétate d'éthyle, on obtient, après séchage et évaporation le produit attendu.

### Exemple 22 : Acide 1-(R)-{[Ac-(R)Phe-(N-cyclopentyl)Gly]amino}-4-[(1-méthyl imidazol-2-yl)thio]butyl boronique, acétate

Le produit attendu est obtenu selon le procédé décrit dans la préparation C à partir du composé décrit dans l'exemple 21.

### Etude pharmacologique des composés de l'invention

### Exemple 23 : Activité anti-coagulante, mesure des temps de thrombine et de prothrombine chez l'homme

En présence d'une quantité standard de thrombine ou de thromboplastine calcique, un plasma normal coagule en un temps défini et constant, appelé respectivement temps de thrombine (TT) et temps de prothrombine (TP). Au pli du coude, le sang veineux est recueilli dans une solution de citrate trisodique (0.109 M). Un plasma pauvre en plaquettes est obtenu par centrifugation des échantillons sanguins (3000 g, 15 minutes).

Le temps de thrombine est réalisé avec le réactif Thrombin Prest (Stago) et le temps de prothrombine avec le réactif Néoplastine (Stago). Ils sont déterminés automatiquement en utilisant un coagulomètre ST4 (Stago). L'antagoniste ou le solvant (10 µl) est additionné au plasma (90 µl), puis incubé 2 minutes à 37°C. 100 µl de thrombine ou 200 µl de thromboplastine calcique sont ajoutés en déclenchant le chronomètre. Dans ces conditions, le TT obtenu dans le plasma témoin est de l'ordre de 20 secondes chez l'homme, et le TP de l'ordre de 12 secondes. L'activité d'un inhibiteur est évaluée par sa capacité à prolonger ces temps par rapport au témoin. Dans ces conditions, les composés de l'invention permettent une prolongation du temps de thrombine et du temps de prothrombine de 50 fois et plus. L'effet des inhibiteurs est mesuré et la concentration qui multiplie par 2 le temps de coagulation (CTT₂ pour le temps de thrombine et CTP₂ pour le temps de prothrombine) est déterminée.

Les résultats sont reproduits dans le tableau suivant :

| Produits | CTP₂ (µM) | CTT₂ (µM) |
|---|---|---|
| Exemple 8 | 3.06 | 0.30 |
| Réf. : DUP 714 | 2.63 | 0.18 |

### Exemple 24 : Inhibition de la thrombine et des sérines protéases de la coagulation et la fibrinolyse

Pour évaluer in vitro l'activité inhibitrice des produits boro-Arginiques sur la thrombine humaine (Sigma, activité spécifique 3220 UNIH/mg), le fibrinogène humain purifié (6 µM, Enzyme Research Laboratories) a été ajouté à une quantité donnée de thrombine (0.7 nM) préalablement incubée avec ou sans l'inhibiteur à tester (20°C, 10 minutes).

Pour évaluer in vitro la sélectivité de ces produits vis-à-vis de différentes sérines protéases de la fibrinolyse et la coagulation, le même protocole a été appliqué à la plasmine humaine purifiée (2 nM, Stago), à la protéine C activée humaine purifiée (2 nM, Stago), au facteur X activé humain purifié (2 nM, Calbiochem), à l'activateur tissulaire du plasminnogène (2 nM, Calbiochem), à l'urokinase humaine purifiée (2 nM, Choay), à la kallikreine plasmatique humaine purifiée (2 nM, Calbiochem) en utilisant pour substrats différents peptides paranitroanilidés : <Glu-Phe-Lys-pNA (0.50 mM, S 2403, Kabi) pour la plasmine, N-Cbo-Arg-Gly-Arg-pNA (0.39 mM, S 2765, Kabi) pour le facteur Xa, <Glu-Pro-Arg-pNA (0.52 mM, S 2366, Kabi) pour la protéine C activée, H-D-Pro-Phe-Arg-pNA (0.45 mM, S 2302, Kabi) pour la kallikreine, H-(D)-Ile-Pro-Arg-pNA (0.48 mM, S 2288, Kabi) pour l'activateur tissulaire du plasminogène et <Glu-Gly-Arg-pNA (0.56 mM, S 2444, Kabi) pour l'urokinase.

Inhibiteurs, enzymes et substrats sont dilués dans le même tampon (tampon phosphate 0.01 mM, pH 7.4, contenant 0.12 M de chlorure de sodium et 0.05 % de sérum albumine bovine) puis distribués dans une microplaque en polystyrène sous un volume de 50 µl.

La fibrine formée par la thrombine ou le paranitronilide libérée par l'action de la sérine protéase sont mesurés spectrophotométriquement à 405 nm après respectivement 5 ou 30 minutes de réaction à 20°C.

Le tableau ci-dessous donne la concentration de composé inhibant 50 % de l'activité enzymatique (CI50) en présence du produit de référence (DUP 714) et du composé de l'exemple 2 par rapport au contrôle sans produit. Les résultats démontrent que le composé de l'exemple 2 inhibe aussi puissamment la thrombine que le DUP 714 mais inhibe beaucoup moins les autres sérine-protéases de la coagulation et de la fibrinolyse que le DUP 714. Les composés des exemples 2, 6 et 8 sont donc des inhibiteurs beaucoup plus sélectifs de la thrombine que le DUP 714.

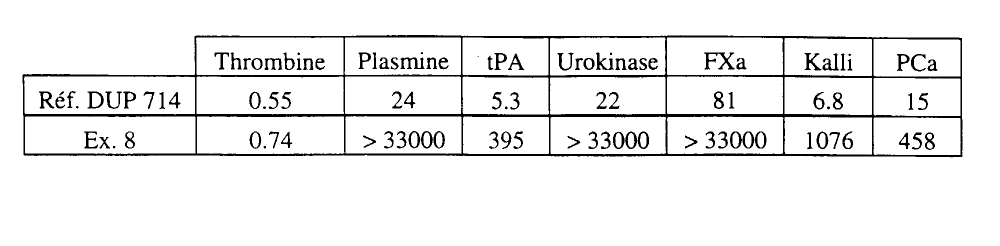

## Revendications

1. Composés de formule (I): 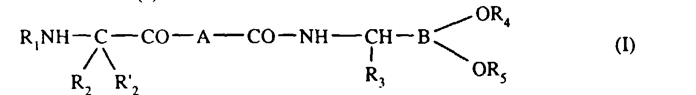 dans laquelle :
R₁ représente un atome d'hydrogène, un groupement acyle (C₁-C₆) linéaire ou ramifié, alkoxycarbonyle (C₁-C₆) linéaire ou ramifié, benzyloxycarbonyle, phénoxycarbonyle, alkyle (C₁-C₆) linéaire ou ramifié (substitué ou non par un ou plusieurs groupements phényle, carboxy, alkoxycarbonyle (C₁-C₆) linéaire ou ramifié, phénoxycarbonyle, benzyloxycarbonyle ou morpholinosulfonyle) ou un groupement R₆SO₂- dans lequel R₆ représente un groupement alkyle (C₁-C₆) linéaire ou ramifié, naphtyle, phényle, benzyle, morpholine (chacun des groupements naphtyle, phényle ou benzyle étant lui-même éventuellement substitué par un ou plusieurs atomes d'halogène ou groupements alkyle (C₁-C₆) linéaire ou ramifié, alkoxy (C₁-C₆) linéaire ou ramifié, trihalogénométhyle, amino, alkylamino ou dialkylamino),
R₂ représente un atome d'hydrogène ou un groupement
- phényle,
- benzyle (substitué ou non sur le noyau phényle par un ou plusieurs atomes d'halogène ou groupements alkyle (C₁-C₆) linéaire ou ramifié, alkoxy (C₁-C₆) linéaire ou ramifié, hydroxy, amino, nitro ou carboxy),
- thiénylméthyle,
- (pyridinyl)méthyle,
- diphénylméthyle,
- fluorényle,
- naphtylméthyle,
- benzocyclobutyle,
- (dicyclopropylméthyl)méthyle,
- indanyle,
- ou, cycloalkyl (C₃-C₇)méthyle,
R'₂ représente un atome d'hydrogène
R₃ représente l'un quelconque des groupements suivants : 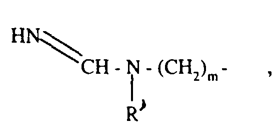 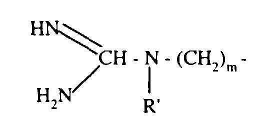 ou, 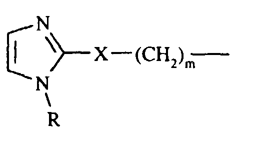 dans lesquels :
1 ≤ m ≤ 6,
R représente un atome d'hydrogène ou un groupement alkyle (C₁-C₆) linéaire ou ramifié,
R' représente un groupement alkyle (C₁-C₆) linéaire ou ramifié,
X représente un atome de soufre ou un groupement amino,
R₄ et R₅ représentent chacun un atome d'hydrogène ou un groupement alkyle (C₁-C₆) linéaire ou ramifié,
ou 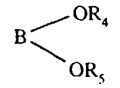 forme un ester boronique de pinanediol,
A représente le groupement suivant: 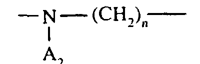 dans lequel :
n représente 1 ou 2,
A₂ représente un groupement phényle, indanyle, cycloalkyle (C₃-C₇) (substitué ou non par un ou plusieurs groupements alkyle (C₁-C₆) linéaire ou ramifié) cycloalkényle (C₃-C₇), bicyclo[2.1.1]hexyl, bicyclo[2.2.1]heptyl ou un groupement : 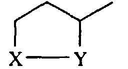 dans lequel
X et Y différents représentent un atome d'oxygène, de soufre, un groupement NH ou CH₂-,
leurs énantiomères, diastéréoisomères et épimères ainsi que leurs sels d'addition à un acide ou à une base pharmaceutiquement acceptable.

2. Composés de formule (I) selon la revendication 1 dans laquelle R₁ représente un groupement acétyl, leurs énantiomères, diastéréoisomères et épimères ainsi que leurs sels d'addition à un acide ou à une base pharmaceutiquement acceptable.

3. Composés de formule (I) selon la revendication 1 dans laquelle R₂ représente un groupement benzyl, leurs énantiomères, diastéréoisomères et épimères ainsi que leurs sels d'addition à un acide ou à une base pharmaceutiquement acceptable.

4. Composés de formule (I) selon la revendication 1 dans laquelle A représente un groupement 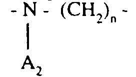 tels que défini dans la revendication 1, leurs énantiomères, diastéréoisomères et épimères ainsi que leurs sels d'addition à un acide ou à une base pharmaceutiquement acceptable.

5. Composé de formule (I) selon la revendication 1 qui est l'acide 1-(R)-{[Ac-(R)Phe-(N-cyclopentyl)Gly]amino}-4-(1N-méthylguanidino)butyl boronique, ses isomères ainsi que ses sels d'addition à un acide ou une base pharmaceutiquement acceptable.

6. Procédé de préparation des composés de formule (I) selon la revendication 1, caractérisé en ce que l'on fait réagir un amino-acide protégé de formule (II), dont on a éventuellement séparé les isomères par une technique classique de séparation : 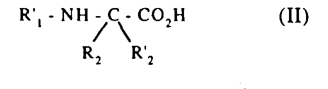 dans laquelle :
R'₁ représente un groupement acyle (C₁-C₆) linéaire ou ramifié, benzyle, alkoxycarbonyle (C₁-C₆) linéaire ou ramifié, benzyloxycarbonyle ou phénoxycarbonyle,
et
R₂ et R'₂ ont la même signification que dans la formule (I),
que l'on fait réagir selon une technique de couplage peptidique avec un deuxième amino-acide protégé de formule (III) dont on a éventuellement séparé les isomères selon une technique classique de séparation,
HA-CO₂CH₂C₆H₅ (III)
dans laquelle A a la même signification que dans la formule (I), pour conduire au composé de formule (IV): 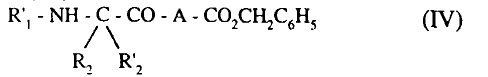 dans laquelle R'₁, R₂, R'₂ et A ont la même signification que précédemment, dont on déprotège la fonction acide par hydrogénation catalytique ou saponification, pour conduire au composé de formule (V): 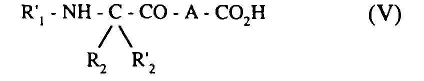 dans laquelle R'₁, R₂, R'₂ et A ont la même signification que précédemment,
que l'on met en réaction avec du N-hydroxysuccinimide en présence de 1,3-dicyclohexylcarbodiimide en milieu anhydre, pour conduire au composé de formule (VI): 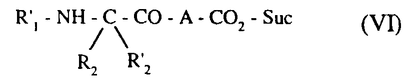 dans laquelle R'₁, R₂, R'₂ et A ont la même signification que précédemment et Suc représente un radical succinimido,
que l'on met en réaction en milieu basique avec un composé de formule (VII): 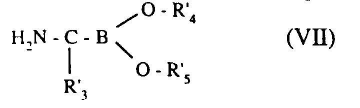 dans laquelle :
R'₃ représente le groupement Br-(CH₂)ₘ- dans lequel m a la même signification que dans la formule (I),
R'₄ et R'₅ représente chacun un groupement alkyle (C₁-C₆) linéaire ou ramifié, ou 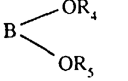 forme un ester boronique de pinanediol,
pour conduire au composé de formule (VIII): 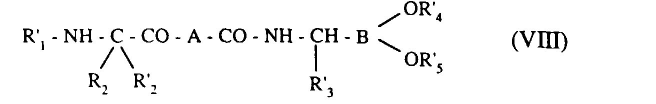 dans laquelle R'₁, R₂, R'₂, R'₃, A, R'₄ et R'₅ ont la même signification que précédemment,
que l'on fait réagir avec de la thiourée éventuellement substituée ou un composé permettant d'accéder au dérivé aminé convenablement substitué,
pour conduire au composé de formule (I/a), cas particulier des composés de formule (I), 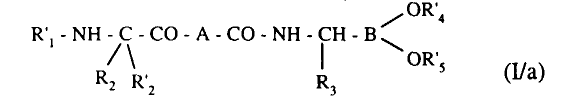 dans laquelle R'₁, R₂, R₃, R'₂, A, R'₄, R'₅, R et m ont la même signification que précédemment,
composé de formule (I/a) dont on déprotège, si on le souhaite, la fonction amine terminale et que l'on transforme, en milieu inerte, à l'aide de trichlorure de bore, en acide boronique de formule (I/b), cas particulier des composés de formule (I): 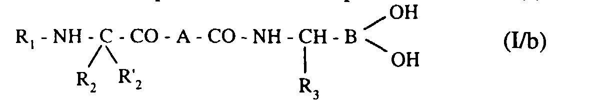 dans laquelle R₁, R₂, R'₂, A et R₃ ont la même signification que dans la formule (I),
composé de formule (I/a) ou (I/b):
- que l'on purifie éventuellement selon une technique classique de purification,
- dont on sépare, si on le souhaite, les énantiomères selon une technique classique de séparation
et que l'on transforme, le cas échéant, en ses sels d'addition à un acide ou une base pharmaceutiquement acceptable.

7. Compositions pharmaceutiques contenant comme principe actif au moins un composé selon l'une quelconques des revendications 1 à 5, seul ou en combinaison avec un ou plusieurs véhicules inertes, non toxiques pharmaceutiquement acceptables.

8. Compositions pharmaceutiques selon la revendication 7 utiles en tant qu'inhibiteurs de trypsine-like serine proteases.

9. Compositions pharmaceutiques selon la revendication 7 utiles en tant qu'inhibiteurs de thrombine.

## Patentansprüche

1. Verbindungen der Formel (I): 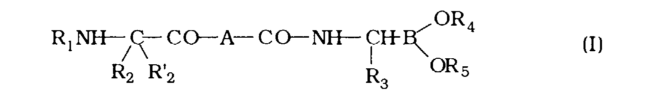 in der:
R₁ ein Wasserstoffatom, eine geradkettige oder verzweigte (C₁-C₆)-Acylgruppe, geradkettige oder verzweigte (C₁-C₆)-Alkoxycarbonylgruppe, Benzyloxycarbonylgruppe, Phenoxycarbonylgruppe, geradkettige oder verzweigte (gegebenenfalls durch eine oder mehrere Phenyl-, Carboxy-, geradkettige oder verzweigte (C₁-C₆)-Alkoxycarbonyl-, Phenoxycarbonyl-, Benzyloxycarbonyl-oder Morpholinosulfonyl-gruppen substituierte) (C₁-C₆)-Alkylgruppe oder eine Gruppe R₆SO₂-, in der R₆ eine geradkettige oder verzweigte (C₁-C₆)-Alkylgruppe, Naphthylgruppe, Phenylgruppe, Benzylgruppe oder Morpholin-gruppe darstellt (wobei die Naphthyl-, Phenyl- oder Benzylgruppen ihrerseits gegebenenfalls durch eines oder mehrere Halogenatome oder geradkettige oder verzweigte (C₁-C₆)-Alkylgruppen, geradkettige oder verzweigte (C₁-C₆)-Alkoxygruppen, Trihalogenmethylgruppen, Aminogruppen, Alkylaminogruppen oder Dialkylaminogruppen substituiert sind),
R₂ ein Wasserstoffatom oder eine:
- Phenylgruppe,
- Benzylgruppe (die am Phenylkern nicht substituiert oder durch ein oder mehrere Halogenatome oder geradkettige oder verzweigte (C₁-C₆)-Alkylgruppen, geradkettige oder verzweigte (C₁-C₆)-Alkoxygruppen, Hydroxygruppen, Aminogruppen, Nitrogruppen oder Carboxygruppen substituiert ist),
- Thienylmethylgruppe,
- (Pyridinyl)-meethylgruppe,
- Diphenylmethylgruppe,
- Fluorenylgruppe,
- Naphthylmethylgruppe,
- Benzocyclobutylgruppe,
- (Dicyclopropylmethyl)-methylgruppe,
- Indanylgruppe,
- oder (C₃-C₇)-Cycloalkyl-methylgruppe
R'₂ ein Wassserstoffatom,
R₃ eine der folgenden Gruppen: 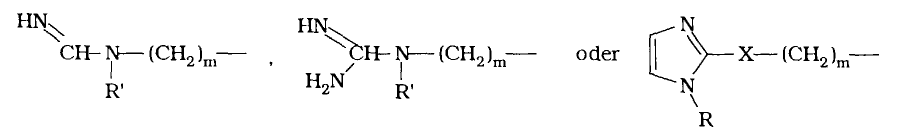 worin :
1 ≤ m ≤ 6,
R ein Wasserstoffatom oder eine geradkettige oder verzweigte (C₁-C₆)-Alkylgruppe,
R' eine geradkettige oder verzweigte (C₁-C₆)-Alkylgruppe und
X ein Schwefelatom oder eine Aminogruppe darstellen,
R₄ und R₅ jeweils ein Wasserstoffatom oder eine geradkettige oder verzweigte (C₁-C₆)-Alkylgruppe, oder 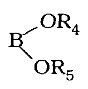 einen Pinandiol-boronsäureester,
A die folgende Gruppe: 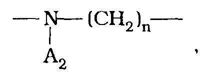 in der
n 1 oder 2,
A2 eine Phenylgruppe, Indanylgruppe, (gegebenenfalls durch eine oder mehrere geradkettige oder verzweigte (C₁-C₆)-Alkylgruppen substituierte) (C₃-C₇)-Cycloalkylgruppe, (C₃-C₇)-Cycloalkenylgruppe, Bicyclo[2.1.1(hexylgruppe, Bicyclo[2.2. 1]heptylgruppe oder eine Gruppe 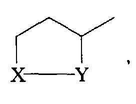 in der X und Y, die voneinander verschieden sind, Sauerstoff, Schwefel, eine NH-Gruppe oder eine Gruppe CH₂- darstellen, bedeuten,
deren Enantiomere, Diastereoisomere und Epimere sowie deren Additionssalze mit einer pharmazeutisch annehmbaren Säure oder Base.

2. Verbindungen der Formel (I) nach Anspruch 1, worin R₁ eine Acetylgruppe darstellt, deren Enantiomere, Diastereoisomere und Epimere sowie deren Additionssalze mit einer pharmazeutisch annehmbaren Säure oder Base.

3. Verbindungen der Formel (I) nach Anspruch 1, worin R₂ eine Benzylgruppe darstellt, deren Enantiomere, Diastereoisomere und Epimere sowie deren Additionssalze mit einer pharmazeutisch annehmbaren Säure oder Base.

4. Verbindungen der Formel (I) nach Anspruch 1, worin A eine Gruppe 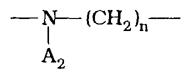 bedeutet, wie sie in Anspruch 1 definiert ist,
deren Enantiomere, Diastereoisomere und Epimere sowie deren Additionssalze mit einer pharmazeutisch annehmbaren Säure oder Base.

5. Verbindung der Formel (I) nach Anspruch 1, nämlich 1-(R)-{[Ac-(R)Phe-(N-Cyclopentyl)-Gly]-amino}-4-(1N-methylguanidino)-butyl-boronsäure und deren Isomere sowie deren Additionssalze mit einer pharmazeutisch annehmbaren Säure oder Base.

6. Verfahren zur Herstellung der Verbindungen der Formel (I) nach Anspruch 1, dadurch gekennzeichnet, daß man eine geschützte Aminosäure der Formel (II), die man gegebenenfalls mit Hilfe klassischer Trennmethoden in ihre Isomeren aufgetrennt hat: 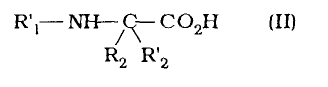 in der:
R'₁ eine geradkettige oder verzweigte (C₁-C₆)-Acylgruppe, Benzylgruppe, geradkettige oder verzweigte (C₁-C₆)-Alkoxycarbonylgruppe, Benzyloxycarbonylgruppe oder Phenoxycarbonylgruppe darstellt
und
R₂ und R'₂ die bezüglich der Formel (I) angegebenen Bedeutungen besitzen, unter Anwendung einer Peptid-Kupplungstechnik mit einer zweiten geschützten Aminosäure der Formel (III), die man gegebenenfalls mit Hilfe klassischer Trennmethoden in ihre Isomeren aufgetrennt hat:
HA - CO₂CH₂C₆H₅ (III)
in der A die bezüglich der Formel (I) angegebenen Bedeutungen besitzt, umsetzt zur Bildung einer Verbindung der Formel (IV): 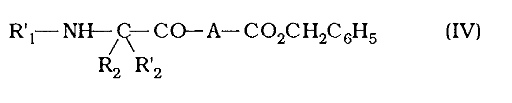 in der R'₁, R₂, R'₂ und A die oben angegebenen Bedeutungen besitzen,
von der man die Säurefunktion durch katalytische Hydrierung oder Verseifung von der Schutzgruppe befreit zur Bildung der Verbindung der Formel (V): 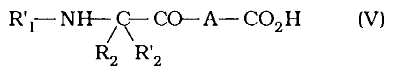 in der R'₁, R₂, R'₂ und A die oben angegebenen Bedeutungen besitzen,
welche man mit N-Hydroxysuccinimid in Gegenwart von 1,3-Dicyclohexylcarbodiimid in wasserfreiem Medium umsetzt zur Bildung der Verbindung der Formel (VI): 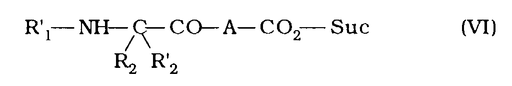 in der R'₁, R₂, R'₂ und A die oben angegebenen Bedeutungen besitzen und Suc einen Succimimidorest darstellt,
welche man in basischem Medium mit einer Verbindung der Formel (VII): 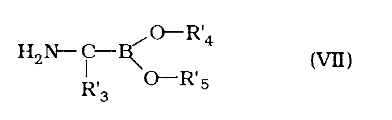 in der:
R'₃ die Gruppe Br-(CH₂)ₘ-, in der m die bezüglich der Formel (I) angegebenen Bedeutungen besitzt und
R'₄ und R'₅ jeweils eine geradkettige oder verzweigte (C₁-C₆)-Alkylgruppe bedeuten oder 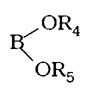 einen Pinandiol-boronsäureester bildet,
umsetzt zur Bildung der Verbindung der Formel (VIII): 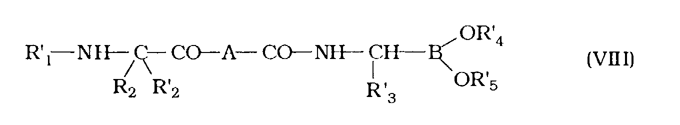 in derR'₁, R₂, R'₂, R'₃, A, R'₄ und R'₅ die oben angegebenen Bedeutungen besitzen,
welche man mit gegebenenfalls substituiertem Thioharnstoff oder einer Verbindung, die einen Zugang zu einem geeignet substituierten Aminoderivat ermöglicht, umsetzt
zur Bildung der Verbindung der Formel (I/a), einem Sonderfall der Verbindungen der Formel (I): 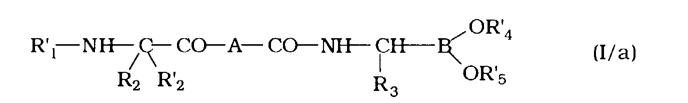 in der R'₁, R₂, R₃, R'₂, A, R'₄, R'₅, R und m die oben angegebenen Bedeutungen besitzen,
von welcher Verbindung der Formel (I/a) man gewünschtenfalls die Schutzgruppe der terminalen Aminofunktion abspaltet und in inertem Medium mit Hilfe von Bortrichlorid in die Boronsäure der Formel (I/b), einem Sonderfall der Verbindungen der Formel (I) umwandelt: 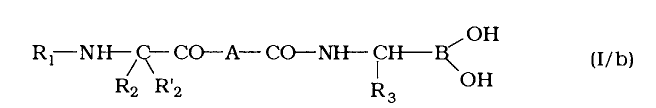 in der R₁, R₂, R'₂, A und R₃ die bezüglich der Formel (I) angegebenen Bedeutungen besitzen,
welche Verbindungen der Formeln (I/a) und (I/b) man:
- gegebenenfalls mit Hilfe einer klassischen Reinigungsmethode reinigt,
- gewünschtenfalls mit Hilfe einer klassischen Trennmethode in ihre Enantiomeren auftrennt
und gegebenenfalls in ihre Additionssalze mit einer pharmazeutisch annehmbaren Säure oder Base umwandelt.

7. Pharmazeutische Zubereitungen enthaltend als Wirkstoff mindestens eine Verbindung nach einem der Ansprüche 1 bi 5 allein oder in Kombination mit einem oder mehreren inerten, nichttoxischen, pharmazeutisch annehmbaren Trägermaterialien.

8. Pharmazeutische Zubereitungen nach Anspruch 7 geeignet als Inhibitoren von Trypsin-artigen Serinproteasen.

9. Pharmazeutische Zubereitungen nach Anspruch 7 geeignet als Thrombin-Inhibitoren.

## Claims

1. Compounds of formula (I): 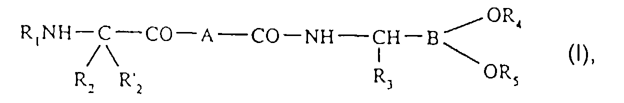 wherein :
R₁ represents a hydrogen atom, a linear or branched (C₁-C₆)acyl group, a linear or branched (C₁-C₆)alkoxycarbonyl group, a benzyloxycarbonyl group, a phenoxycarbonyl group, a linear or branched (C₁-C₆)alkyl group (unsubstituted or substituted by one or more phenyl, carboxy, linear or branched (C₁-C₆)alkoxycarbonyl, phenoxycarbonyl, benzyloxycarbonyl or morpholinosulphonyl groups) or a group R₆SO₂- wherein R₆ represents a linear or branched (C₁-C₆)alkyl group, a naphthyl group, a phenyl group, a benzyl group or a morpholine group (each of the naphthyl, phenyl or benzyl groups itself being optionally substituted by one or more halogen atoms or linear or branched (C₁-C₆)alkyl, linear or branched (C₁-C₆)alkoxy, trihalomethyl, amino, alkylamino or dialkylamino groups),
R₂ represents a hydrogen atom or a group :
- phenyl,
- benzyl (unsubstituted or substituted on the phenyl ring by one or more halogen atoms or linear or branched (C₁-C₆)alkyl, linear or branched (C₁-C₆)-alkoxy, hydroxy, amino, nitro or carboxy groups),
- thienylmethyl,
- (pyridyl)methyl,
- diphenylmethyl,
- fluorenyl,
- naphthylmethyl,
- benzocyclobutyl,
- (dicyclopropylmethyl)methyl,
- indanyl
- or (C₃-C₇)cycloalkylmethyl,
R'₂ represents a hydrogen atom,
R₃ represents any one of the following groups : 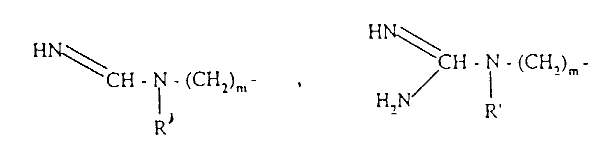 or 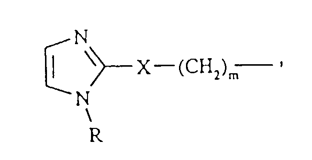 wherein :
1 ≤ m ≤ 6,
R represents a hydrogen atom or a linear or branched (C₁-C₆)alkyl group,
R' represents a linear or branched (C₁-C₆)alkyl group,
X represents a sulphur atom or an amino group,
R₄ and R₅ each represents a hydrogen atom or a linear or branched (C₁-C₆)alkyl group, or 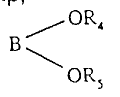 is a pinanediol boronic ester,
A represents the following group : 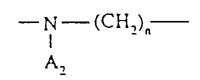 wherein : n is 1 or 2,
A₂ represents a phenyl group, an indanyl group, a (C₃-C₇)cycloalkyl group (unsubstituted or substituted by one or more linear or branched (C₁-C₆)alkyl groups), a (C₃-C₇)cycloalkenyl group, a bicyclo[2.1.1]hexyl group, a bicyclo[2.2.1]heptyl group or a group : 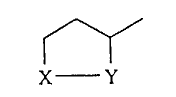 wherein X and Y, which are different, represent an oxygen atom, a sulphur atom, an NH group or a CH₂- group,
their enantiomers, diastereoisomers and epimers, and also addition salts thereof with a pharmaceutically acceptable acid or base.

2. Compounds of formula (I) according to claim 1, wherein R₁ represents an acetyl group, their enantiomers, diastereoisomers and epimers, and also addition salts thereof with a pharmaceutically acceptable acid or base.

3. Compounds of formula (I) according to claim 1, wherein R₂ represents a benzyl group, their enantiomers, diastereoisomers and epimers, and also addition salts thereof with a pharmaceutically acceptable acid or base.

4. Compounds of formula (I) according to claim 1, wherein A represents a group 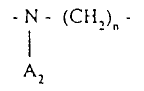 as defined in claim 1, their enantiomers, diastereoisomers and epimers, and also addition salts thereof with a pharmaceutically acceptable acid or base.

5. Compound of formula (I) according to claim 1, which is 1-(R)-{[Ac-(R)Phe-(N-cyclopentyl)Gly]amino}-4-(1 N-methylguanidino)butylboronic acid, its isomers, and also addition salts thereof with a pharmaceutically acceptable acid or base.

6. Process for the preparation of compounds of formula (I) according to claim 1, characterised in that a protected amino acid of formula (II), the isomers of which have been separated, if desired, by a conventional separation technique : 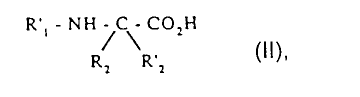 wherein :
R'₁ represents a linear or branched (C₁-C₆)acyl group, a benzyl group, a linear or branched (C₁-C₆)alkoxycarbonyl group, a benzyloxycarbonyl group or a phenoxycarbonyl group,
and
R₂ and R'₂ are as defined for formula (I)_{,}
is reacted according to a peptide coupling technique with a second protected amino acid of formula (III), the isomers of which have been separated, if desired, by a conventional separation technique :
HA-CO₂CH₂C₆H₅ (III),
wherein A is as defined for formula (I), to yield the compound of formula (IV): 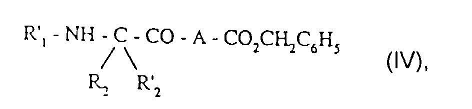 wherein R'₁, R₂, R'₂ and A are as defined hereinbefore,
the acid function of which is deprotected by catalytic hydrogenation or hydrolysis to yield the compound of formula (V): 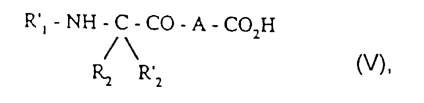 wherein R'₁, R₂, R'₂ and A are as defined hereinbefore,
which is reacted with N-hydroxysuccinimide in the presence of 1,3-dicyclohexylcarbodiimide in an anhydrous medium to yield the compound of formula (VI): 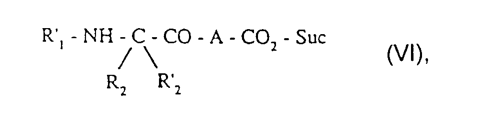 wherein R'₁, R₂, R'₂ and A are as defined hereinbefore and Suc represents a succinimido radical,
which is reacted in a basic medium with a compound of formula (VII): 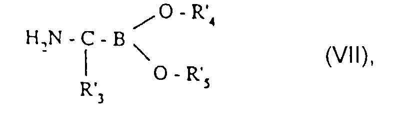 wherein :
R'₃ represents the group Br-(CH₂)ₘ- wherein m is as defined for formula (I),
R'₄ and R'₅ each represents a linear or branched (C₁-C₆)alkyl group, or 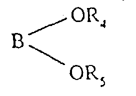 is a pinanediol boronic ester, to yield the compound of formula (VIII): 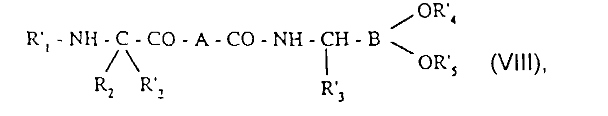 wherein R'₁, R₂, R'₂, R'₃, A, R'₄ and R'₅ are as defined hereinbefore,
which is reacted with optionally substituted thiourea or a compound allowing production of the appropriately substituted aminated compound
to yield the compound of formula (I/a), a particular case of the compounds of formula (I), 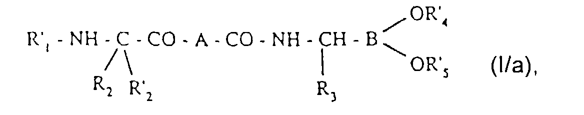 wherein R'₁, R₂, R₃, R'₂, A, R'₄, R'₅, R and m are as defined hereinbefore,
which compound of formula (I/a) is, if desired, deprotected at the terminal amine function and is converted, in an inert medium, using boron trichloride, into the boronic acid of formula (I/b), a particular case of the compounds of formula (I): 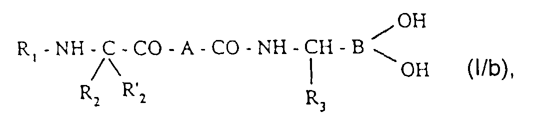 wherein R₁, R₂, R'₂, A and R₃ are as defined for formula (I),
which compound of formula (I/a) or (I/b):
- is purified, if necessary, according to a conventional purification technique,
- is separated, if desired, into its enantiomers according to a conventional separation technique,
and is converted, if appropriate, into its addition salts with a pharmaceutically acceptable acid or base.

7. Pharmaceutical compositions comprising as active ingredient at least one compound according to any one of claims 1 to 5 alone or in combination with one or more pharmaceutically acceptable, inert, non-toxic carriers.

8. Pharmaceutical compositions according to claim 7 for use as inhibitors of trypsin-like serine proteases.

9. Pharmaceutical compositions according to claim 7 for use as thrombin inhibitors.
